# EUROPEAN PATENT SPECIFICATION

(11) **EP 1 890 710 B1**
(45) Date of publication and mention of the grant of the patent: **02.11.2011**
(21) Application number: 06742888.8
(22) Date of filing: 12.05.2006
(51) Int. Cl.: A61K 31/7008

(54) **PRODUCTION OF GLUCOSAMINE FROM PLANT SPECIES**
HERSTELLUNG VON GLUCOSAMIN AUS PFLANZENSPEZIES
PRODUCTION DE GLUCOSAMINE A PARTIR DE PLANTES

(30) Priority: 13.05.2005 EP 05104038
(43) Date of publication of application: 27.02.2008
(73) Proprietor: Nestec S.A., 1800 Vevey (CH)
(72) Inventor: COURTOIS, Didier, F-37550 St-Avertin (FR); MICHAUX, Stéphane, F-37380 Monnaie (FR); GOULOIS, Eric, F-37530 Charge (FR)
(86) International application number: PCT/EP2006/004472
(87) International publication number: WO 2006/120009

(56) References cited:
- EP-A- 1 172 041
- WO-A-00/74696
- US-B1- 6 413 525

## Description

The present invention relates to a process leading to raw plant materials containing levels of glucosamine equal or higher than 0.5% (wt) of the plant dry matter.

### Background of the Invention

### Use of glucosamine

The use of pure glucosamine in the treatment of joint diseases is widely described in the patent as well as in the scientific literature, usually in combination with other compounds or extracts from various natural sources. Pure glucosamine is added as glucosamine hydrochloride or glucosamine sulphate, and comes from shellfish hydrolysis. For example, WO2000/0074696 describes "herbal compositions comprising glucosamine and *Trypterygium wilfordii, Ligustrum lucidum* and/or *Erycibe schmidtii,* for treating inflammation or degeneration of joint tissues, e.g. arthritis" where pure glucosamine is mixed with plant preparation. Other patents relate to compositions of plant carbohydrates as dietary supplements (EP 1 172 041 or EP 923 382) where glucosamine is originated from chitin, i.e. once again from shellfish hydrolysis.

The use of glucosamine as an anti-osteoarthritis agent has been intensively developed during the last decade. Glucosamine is suspected to be the sole active compound on joint disease such as osteoarthritis (up to recently only symptomatic treatment such as non-steroidal anti-inflammatory drugs have been sought to be efficient).

Glucosamine has also been shown to prevent the cartilage degradation by inhibiting the production of MMPs (Matrix MetalloProteases) such as MMP1, MMP3 and MMP13.
Interestingly glucosamine is also related to the aging process of skin, which has been characterized mainly by the continuous loss of elasticity and the loss of moisture of said skin. Skin aging is reflected by major structural changes and variations in composition. Most notably aged skins have less collagen and glycosaminoglycans compared with young skins. Glycosaminoglycan molecules produced by the skin include hyaluronic acid (poly d-glucuronic acid-n-acetyl-d-glucosamine), chondroitin sulfate, and dermatan sulfate. Hyaluronic acid is produced in higher quantities by the skin cells in response to exfoliation. Hyaluronic acid has a large capacity for hydration.

Inhibiting MMP-1 is related to the inhibition of the polyglycan/collagen degradation, and therefore also related to skin ageing: MMP-1 can be induced by UV and is recognized as a marker of the skin ageing. In US 2002/119107, the invention is based on the selective inhibition of MMP-1 claiming topical compositions for protecting human skin from collagen degradation. US 2004/037901 claims a regime for inhibiting the adverse signs of effects of cutaneous aging comprising an extract from rosemary plant inhibiting the expression of metalloproteases.

Glucosamine has been shown to significantly improve dryness of the skin and exfoliation. Glucosamine increases the moisture content and improves the smoothness of the skin. These findings suggest that long-term intake of glucosamine is effective in improving moisture content and smoothness of the skin.

It has been shown that oral supplement containing glucosamine lead to a reduction (34%) in the number of visible wrinkles and (34%) in the number of fine lines in a group of women who took the supplement. The use of an oral supplement containing glucosamine, minerals, and various antioxidant compounds can potentially improve the appearance of visible wrinkles and fine lines.

US 6 413 525 describes methods of substantially exfoliating the skin. In particular, the invention relates to topically applied compositions containing an amino sugar in the form of N-acetyl glucosamine: when young skin cells are exposed after exfoliation, they produce larger quantities of hyaluronic acid which is a glycosaminoglycan composed of a chain of alternating, repeating, D-glucuronic acid and N-acetyl-D-glucosamine molecules. N-acetyl-D-glucosamine is known to be a rate-limiting factor in the hyaluronic acid production by living cells. The topical application of glucosamine assists in the continued production of hyaluronic acid.

Other Compositions for topical application containing N-acetyl-D-glucosamine have also been disclosed for example, in JP 59 013 708 (soften and moisturize the skin) or U.S. Pat. No. 5 866 142 (a composition for exfoliating the skin).

### Origin of glucosamine

Glucosamine, 2-amino-2-deoxy-D-glucose, is a naturally occurring derivative of fructose and is an essential component of glycoproteins and proteoglycans, important constituents of many eukaryotic proteins. This is an essential component of mucopolysaccharides and chitin. Glycosaminoglycans (mucopolysaccharides) are large complexes incorporated into connective tissue, skin, tendons, ligaments and cartilage.

### Industrial sources of glucosamine

Industrial glucosamine is a pure compound obtained from the acidic hydrolysis of chitin from shellfish, a complex carbohydrate derived from N-acetyl-D-glucosamine. As an example, US patent 6 486 307 describes an improved method for chitin acidic hydrolysis: a method of producing glucosamine hydrochloride from chitin by grinding the chitin to a very fine size and digesting it with concentrated hydrochloric acid.

Glucosamine can also be produced from enzymatic hydrolysis of shellfish. As an example, US patent 5 998 173 describes a novel process for directly producing N-acetyl-D-glucosamine from chitin utilizing an ensemble of the chitinase family of enzymes to hydrolyze chitin of crustacean shells.

Patents have also been filed protecting microbial fermentation processes where cultivated microorganisms biosynthesize glucosamine. As an example, US 6 372 457 describes a method and material for producing glucosamine by fermentation using a genetically modified microorganism.

All these processes concern the production of pure, extracted glucosamine, in competition with shellfish extracts.

In WO2005/053710, it has been found that glucosamine can be formed from several raw plant materials by following a special drying process, therefore obtaining glucosamine contents of between 150 and 1000mg per kg dry weight.

### Summary of the invention

In a first aspect, the present invention describes new processes to obtain glucosamine from plants by adding a glucosamine precursor after harvesting the plant material and before or during a heating process in order to obtain plant raw materials containing level of glucosamine higher than 0.5% (5g per kg dry weight) of the dry matter. The present invention therefore allows reaching glucosamine content in plant material much higher than previously described in the prior art, for example reflected by W02005/053710. The consequence is that a less raw plant material or plant extract is required to reach the active dose of glucosamine described in the literature. Therefore, the process is more usable at an industrial scale.

The above-mentioned precursors can be added after harvesting on fresh or previously dried plant material or on derived-plant extract, during the heating process of the plant material or derived-plant extract, or during the preparation of an aqueous extract from plant material.

### Detailed Description of the Invention

In the present specification, the word "heating" (and derived "heated") has to be understood as a heating process in the range of temperature of 70-110°C, for more than 10 hours and preferably for less than one week. This heating process can be described as a drying process. The heating process can also consist in a liquid maceration, taking place at the same temperature and time conditions, replacing the drying process.

In the present specification, by "free glucosamine", it has to be understood non-polymerized glucosamine.

In the present specification, by "high amount of glucosamine" it has to be understood that the amount of glucosamine is higher than traces of glucosamine, higher than the amounts in the corresponding fresh (non-dried) material and higher than any content cited in literature or patents. It should be understood as glucosamine present in amounts above 5g per kg dry matter of plant raw material, preferably above 20g per kg dry matter of plant raw material, and most preferably above 40g per kg dry matter of plant raw material.

In the present specification, "plant" and "plant material" are considered as synonyms. By "plant, "plant material" or "plant extract" it has to be understood any plant material capable of generating glucosamine according to the heating process of the invention, and any type of plant extract obtained by any extraction procedure known to the skilled person from said plant material capable of generating glucosamine according to the heating process of the invention. For example, a plant comprising a certain amount of glucosamine can be a dried or rehydrated plant material having undergone the process of the invention. A plant extract comprising a certain amount of glucosamine can be an aqueous solution extracted from said plant having undergone the process of the invention.

With respect to the first object of the present invention, the plant or plant extract are processed according to the invention in order to contain natural free glucosamine in high amount.

In a preferred embodiment, the plant or plant extract is from any part of the plant, e.g. leaves, tubers, fruits, seeds, roots, grains or cell cultures. After a controlled heating process of the plant raw material, the plant or plant extract may be in the form of a dried, lyophilized extract of leaves, roots and/or fruits depending on the source of plant, or fresh plant, or glucosamine-enriched fraction.

The plant or plant extract is selected for its ability to generate free glucosamine through the process of the present invention; in particular it may be selected from the group consisting of plant species containing sucrose, fructose or inulin such as *Cichorium, Daucus, Helianthus, Beta.*

In a most preferred embodiment the plant material or plant extract may be for example from root of Chicory (*Cichorium intybus*), carrot (*Daucus carota*), tuber of Jerusalem artichoke (*Helianthus tuberosum*), root of beet *(Beta vulgaris).*

In one embodiment, fresh plant material or plant material can be first dried completely or partially, then subsequently re-hydrated and after these two steps can be processed according to the new invention to obtain plant material with high glucosamine content.

In a preferred embodiment, fresh plant material is used.

As disclosed in WO2005/053710, the drying process described is one way to obtain glucosamine in plants in large amounts: levels around 500 mg per kg dry matter of chicory root, 100 mg per kg dry matter of carrot root, or 50mg per kg dry matter of Jerusalem artichoke tubers or beet root can be obtained using the drying process described in WO2005/053710.

Fresh or dried or re-hydrated plant raw material are heated using liquid maceration or drying process at a temperature below 110°C, preferably at temperatures comprised between 70 and 110°C, most preferably between 70 and 91 °C or below for more than 10 hours and preferably less than one week, preferably between 10 and 120 hours, for example between 12 and 50 h, depending on the plant species and plant organ. If the temperatures and/or heating times are too low and/or too short, the generation of glucosamine won't be efficient or will be very slow, leading to a process that will not be economically viable. On the contrary, if the temperatures and/or heating time are too high and/or too long, the glucosamine will be generated but subsequently progressively degraded.

Therefore, the temperatures and times are chosen in order to obtain glucosamine contents of at least 5 g glucosamine /kg of dry matter of the corresponding plant material having undergone the heating process.

A most preferred example comprises a drying in an oven at a temperature of 85°C between 48 and 72 hours.

According to the present invention, the same process is used but the difference lies in the fact that plant materials or plant extracts are first put in contact with a precursor of glucosamine. The result is that the obtained quantities of glucosamine are much more higher than in WO 2005/053710. Indeed, the glucosamine content of plants according to the present invention is higher than 10g per kg dry matter of chicory root, than 15g per kg dry matter of carrot root or beetroot.

The precursors of glucosamine used according to the present invention are compounds allowing the formation of the sugar-nitrogen compound condensation required to form glucosamine. Preferably, they consist in ammonium salts. Examples of such ammonium salts are ammonium nitrate, ammonium sulfate, ammonium acetate, ammonium dihydrogenophosphate or glutamine, among others. The preferred precursors of glucosamine are ammonium sulfate and ammonium nitrate that have shown surprisingly good results in the process according to the invention.

In a preferred embodiment, the precursors are added on the fresh harvested material shortly before the heating process. The skilled person will know how to adopt the quantity of glucosamine precursor on the plant material or the plant extract, however, in a most preferred embodiment, the final quantity of the ammonium sulfate is between 1 and 8% of fresh weight of plant raw material, preferentially 4%.

In a most preferred embodiment, the glucosamine precursor is added to the plant material or the plant extract as a solution which is applied through spraying or by soaking. For example spraying of 200ml of an aqueous solution (4M) between 5 and 30 minutes, or soaking in the same solution for few minutes to several hours. these examples are not to be considered in any way limitative of the invention. That is, the skilled person will recognize many variations in this example to cover a wide range of processing, and mixtures to rationally adjust the naturally occurring levels of the compounds of the invention for a variety of applications.

For the final process leading to glucosamine formation, a suitable process is described in WO 2005/053710 for the preparation of the plant material without added precursors: the plant material is harvested, cut and dried in an oven or in an industrial dryer at a temperature below 110°C, preferably between 80 and 105°C, most preferably 91 °C or below for more than 10 hours and preferably less than one week, preferably between 10 and 120 hours, for example between 12 and 50 h, depending on the plant species and plant organ. Although not wishing to be bound by theory, we believe that it is preferable to cut the plant material, for example in slices or dices, preferably having a maximum width of 5 mm. The inventors indeed believe that it is important for the present invention in order to reach optimized thermodynamic exchanges.

The addition of glucosamine precursors after the harvest, before or during the heating process allows to significantly increase the above-described reaction, from a few hundred mg of glucosamine per kg dry weight without precursor to at least 5g glucosamine per kg dry matter of the corresponding plant material.

The process of the present invention generates glucosamine directly in free form. Without wishing to be bound by theory, it is believed that at least half of the glucosamine produced by said process is in free form, and even that almost all the glucosamine produced is in free form. Indeed, it is believed that at least 50%, at least 70%, and even at least 90% of the glucosamine is produced in free form according to the process of the invention. This is another advantage of the present invention compared to known techniques used to produce glucosamine, wherein an hydrolysis step is mandatory to release free glucosamine from complex molecules such as chitin, glycoproteins or proteoglycans, for example.

A pre-extraction of the raw material with ammonium sulfate before the heating treatment leads to enriched extracts in glucosamine (example 8).

Glucosamine maybe first extracted from the plant material, alone or jointly with other compounds such as inulin or fructooligosaccharides (FOS).

### Examples

The following examples are illustrative of some of the products and methods of making the same falling within the scope of the present invention. They are not to be considered in any way limitative of the invention. Changes and modifications can be made with respect to the invention. That is, the skilled person will recognize many variations in these examples to cover a wide range of formulas, ingredients, processing, and mixtures to rationally adjust the naturally occurring levels of the compounds of the invention for a variety of applications.

### Example 1: Fresh Sugar beet

Drying: After harvest, 200g (fresh weight) roots of beet (*Beta vulgaris*) are cut in dices of 1x1x1cm. 25 ml Solution of ammonium sulfate (NH4)₂SO₄, 4M (Prolabo ref:21332.293), are sprayed on all faces of the dices and then the dices are dried in an oven at a temperature of 91 °C for 56 h.

### Analysis:

### Extraction of glucosamine:

2g of ground and specifically dried beetroot are extracted with 20ml of water at room temperature for 1 minute. The solution is filtered on filter Schleicher & Schultz (n°597) or centrifuged. A purification step of the solution is performed using a cation exchange column (Oasis cartridge WATERS, MCX type, ref. 186 000 776). Basic compounds entrapped on the matrix are eluted with MeOH/NH₄OH 2% (v/v). After filtration, an aliquot is used for direct injection on LC system (DIONEX).

### Separation:

Analysis is carried out with a HPAE/PED system using an ion exchange PA1 column (4*250mm) with DIONEX DX 500 apparatus.

### Programme:

ELUTION (%)

| Time (min) | H₂O | 0.1M NaOH | 0.25 NaOH | Comment |
|---|---|---|---|---|
| 0 | 85 | 15 | 0 | Balancing |
| 60 | 85 | 15 | 0 | |
| 60.1 | 0 | 0 | 100 | Washing |
| 70 | 0 | 0 | 100 | |
| 70.1 | 85 | 15 | 0 | Balancing |
| 90 | 85 | 15 | 0 | |

Flow: 1ml/min. Volume of injection: 20µl. Standard: Glucosamine from Sigma (ref: G4875).

In these conditions, glucosamine has a retention time of round 11 min and is easily detected for further quantification in beet extracts properly processed. A concentration of 16g/kg dry weight has been quantified by this method in the present example, instead of less than 300mg/kg without precursor and less than 1 mg/kg without heating process. Confirmation of the presence of glucosamine:
In order to confirm the presence of glucosamine in beet plant extracts, three different qualitative techniques have been evaluated.

### Thin layer chromatography (TLC)

Pure glucosamine and plant extracts were analyzed on HPTLC (High Performance Thin Layer Chromatography) silica gel plates (Merck, ref. 1.05642) with Ethyl acetate/MeOH/water (50/50/10; V/V/V) as eluant. After elution, the plates are sprayed with an acetic acid solution of ninhydrine 1% and heated at 120°C for 10min. One spot appeared in a pink/blue color at the same rate factor (Rf) for the reference and extracts.

### Chemical degradation

In the presence of ninhydrine, an oxidative de-amination occurs with glucosamine, which leads to the release of arabinose easily detected through routine sugar LC analysis. Presence of arabinose with control and chicory extracts was unambiguously confirmed.

### Derivatization of glucosamine

Reverse phase chromatography using pre-column derivatization with phenylisothiocyanate and UV detection (λ=254nm) was used with the pure compound and plant extracts as described by Zhongming et al.: "Determination of nutraceuticals, glucosamine hydrochloride in raw materials, dosage form and plasma using pre-column derivation with UV HPLC. In J. of Pharmaceut. and Biomed. Analysis, 1999 (20), 807-814."
The corresponding peak of derivatized glucosamine was detected in chicory extracts as well as with pure compound.

### Mass spectrum analysis

Plant extracts were analyzed by Electrospray Mass Spectrometry in positive ionization mode to confirm the presence of glucosamine. The mass spectrometer was a time-of-flight instrument (LCT from Micromass with a Z-spray interface). Standard glucosamine give an ion at m/z 180.0887. This ion fragment is found in analyzed plant extracts.

### Example 2: Fresh roots of carrot (Daucus carota)

200g (fresh weight) of carrot roots are cut in dices of 1x1x1cm then solution of ammonium sulfate as in example 1 is sprayed. Then the dices are dried in an oven at a temperature of 91°C for 37 h. Extraction and analysis are performed as in example 1, leading to a glucosamine concentration of 15g/kg dry weight, instead of less than 190mg / kg dry weight without precursor and less than 1mg / kg without heating process.

### Example 3: Fresh roots of chicory (Cichorium inybus)

200g (fresh weight) of chicory roots are cut in dices of 0.5x0.5x0.5cm. Dices are soaked in a solution of ammonium sulfate (4M) for 30 minutes, or the solution of ammonium sulfate is sprayed, or dices are mixed at 85°C with the solution of ammonium sulfate for 8h. Then the dices are dried in an oven at a temperature of 91 °C for 40 h. Extraction and analysis are performed as in example 1, leading to a glucosamine concentration of 43g / kg dry weight, instead of less than 900mg/kg dry weight without precursor and less than 10mg/kg without heating process or in commercial dried roots chicory.

### Example 4: Dried roots of chicory

Fresh chicory roots are cut in dices (1x1x1cm), dried using a current procedure (115°C air inlet for 2 hours in a fluidized bed dryer) then stored at room temperature. When glucosamine generation is needed, the dried dices are soaked 1 hour in a solution of ammonium sulfate (4M), re-hydrating the dices and bringing the precursor. Then the dices are dried in an oven at a temperature of 85°C for 43hours. Extraction and analysis are performed as in example 1, leading to a glucosamine concentration of 11g/kg dry weight, instead of less than 190mg / kg dry weight without precursor and less than 1mg/kg without heating process.
It is therefore possible to apply the present invention to plant materials submitted to a long-term storage under dried form.

### Example 5: Dried powder roots of chicory

1.5 g of dried powder is suspended in an Erlenmeyer flask with 100ml of ammonium sulfate (3M), shaken vigorously and incubated for 30 minutes at 80°C. Then this solution is dried in an oven at a temperature of 85°C for 50 h. Extraction and analysis are performed as in example 1, leading to a glucosamine concentration of 110 g/kg dry weight.
It is another example that it is therefore possible to apply the present invention to plant materials submitted to a long-term storage under dried form.

### Example 6: Entire dried roots of chicory

10 entire roots of chicory freshly harvested are stored at ambient temperature for three days. The entire roots are then soaked in a solution of ammonium sulfate (4M) for 24 hours. Then the roots are cut in dices of 0.5x0.5x 0.5cm then processed (dried) as in example 3, leading to a glucosamine concentration of 44 g/kg dry weight.

### Example 7: Dices of fresh roots of chicory treated in liquid medium

100g of fresh chicory roots are cut in dices (0.5x0.5x0.5cm), suspended and shaken vigorously in an Erlenmeyer flask, opened or corked with a cellulose plug, with 200ml of ammonium sulfate (4M). The solution is then heated in an oven at a temperature of 85°C for 60h. Extraction and analysis are performed as in example 1 leading to a glucosamine concentration of 30g/kg fresh weight. It is therefore also possible to apply the present invention under wet conditions of heating.

### Example 8: Enriched extract in glucosamine

100g of fresh chicory roots are cut in dices (0.5x0.5x0.5cm), suspended and shaken vigorously in an Erlenmeyer flask with 200ml of ammonium sulfate (4M). Then the solution is filtered and the obtained eluant is dried in an oven at a temperature of 85°C for 60h. Extraction and analysis are performed as in example 1 leading to a glucosamine concentration of 100g/kg dry weight.

## Claims

1. A process for generating glucosamine from plants wherein fresh plant materials, or re-hydrated dried plant materials or plant extracts, are heated at a temperature comprised between 70 and 110°C for more than 10 hours, **characterized in that** a glucosamine precursor is added to said plant materials, re-hydrated plant materials or plant extract,
wherein the glucosamine precursors are compounds allowing the formation of the sugar-nitrogen compound condensation required to form glucosamine, and are preferably ammonium salts.

2. A process according to claim 1 wherein the glucosamine precursor is added before the heating step.

3. A process according to one of claims 1 to 2 wherein the glucosamine precursor is added or is also added during the heating step.

4. A process according to one of claims 1 to 3 wherein the plant species belongs to the genus *Cichorium, Daucus, Heliantus, or Beta,*

5. A process according to one of claims 1 to 4 wherein the plants are chicory (*Cichorium intybus*), carrot (*Daucus carota*), Jerusalem artichoke (*Helianthus* tuberosum), and/or beet *(Beta vulgaris).*

6. A process according to one of claims 1 to 5 wherein the plant or plant extract comprises at least 5g glucosamine/kg dry weight of plant material, preferably above 20g/kg dry matter, and most preferably above 40g/ kg dry matter.

## Patentansprüche

1. Verfahren zur Erzeugung von Glucosamin aus Pflanzen, bei dem frische Pflanzenmaterialien oder rehydrierte getrocknete Pflanzenmaterialien oder Pflanzenextrakte bei einer Temperatur zwischen 70 und 100°C für mehr als 10 Stunden erhitzt werden, **dadurch gekennzeichnet, dass** ein Glucosaminprecursor zu diesen Pflanzenmaterialien, rehydrierten Pflanzenmaterialien oder Pflanzenextrakten gegeben wird, wobei die Glucosaminprecursor Verbindungen sind, die die Entstehung der zur Bildung von Glucosamin erforderlichen Zucker-Stickstoffverbindungs-Kondensation erlauben, und vorzugsweise Ammoniumsalze sind.

2. Verfahren nach Anspruch 1, wobei der Glucosaminprecursor vor dem Erhitzungsschritt zugegeben wird.

3. Verfahren nach einem der Ansprüche 1 bis 2, wobei der Glucosaminprecursor während des Erhitzungsschritts oder auch während des Erhitzungsschritts zugegeben wird.

4. Verfahren nach einem der Ansprüche 1 bis 3, wobei die Pflanzenart zur den Gattungen *Cichorium, Daucus, Helianthus* oder *Beta* gehört.

5. Verfahren nach einem der Ansprüche 1 bis 4, wobei die Pflanzen Chicorée (*Cichorium intybus*), Karotte (*Daucus carota*), Topinambur (*Helianthus tuberosum*) und/oder Rübe (*Beta vulgaris*) sind.

6. Verfahren nach einem der Ansprüche 1 bis 5, wobei die Pflanze oder der Pflanzenextrakt mindestens 5g Glucosamin/kg Trockengewicht des Pflanzenmaterials, vorzugsweise über 20g/kg Trockenmasse und am meisten bevorzugt über 40g/kg Trockenmasse enthält.

## Revendications

1. Un procédé de production de glucosamine à partir de plantes, dans lequel des matières végétales fraîches, ou des matières végétales sèches réhydratées ou des extraits de plantes, sont chauffés à une température comprise entre 70 et 110°C pendant plus de 10 heures, **caractérisé en ce qu'**un précurseur de glucosamine est ajouté auxdits matières végétales, matières végétales réhydratées ou extraits de plantes, dans lequel les précurseurs de glucosamine sont des composés permettant la formation de la condensation de composé(s) sucre-azote requise pour former de la glucosamine, et sont de préférence des sels d'ammonium.

2. Un procédé selon la revendication 1 dans lequel le précurseur de glucosamine est ajouté avant l'étape de chauffage.

3. Un procédé selon la revendication 1 ou 2 dans lequel le précurseur de glucosamine est ajouté ou est aussi ajouté pendant l'étape de chauffage.

4. Un procédé selon l'une des revendications 1 à 3 dans lequel les espèces végétales appartiennent au genre *Cichorium, Daucus, Helianthus,* ou *Beta.*

5. Un procédé selon l'une des revendications 1 à 4 dans lequel les plantes sont de la chicorée (*Cichorium intybus*), de la carotte (*Daucus Carota*), du topinambour (*Helianthus tuberosum*) et/ou de la betterave *(Beta vulgaris*)*.*

6. Un procédé selon l'une des revendications 1 à 5 dans lequel la plante ou l'extrait de plante comprend au moins 5g de glucosamine par kg en poids sec de matière végétale, de préférence plus de 20 g/kg de matière sèche, et encore plus préférablement plus de 40 g/kg de matière sèche.
